# EUROPÄISCHE PATENTANMELDUNG

(11) **EP 1 188 455 A1**
(43) Veröffentlichungstag der Anmeldung: **20.03.2002**
(21) Anmeldenummer: 00120455.1
(22) Anmeldetag: 19.09.2000
(51) Int. Cl.: A61M 5/24, A61M 5/19, B01F 13/00

(54) **Vorrichtung zum Austragen des Inhalts eines dicht abgeschlossenen, flüssigkeits-, pasten- oder gasgefüllten Behältnisses**

(71) Anmelder: TRANSCOJECT GESELLSCHAFT FÜR MEDIZINISCHE GERÄTE mbH & CO. KG, 24539 Neumünster (DE)
(72) Erfinder: Rolle, Philipp, 24534 Neumünster (DE)
(74) Vertreter: Vollmann, Heiko, Dipl.-Ing.

(57) **Zusammenfassung**

Die Vorrichtung weist ein dicht abgeschlossenes flüssigkeits-, pasten- oder gasgefülltes Behältnis auf sowie Mittel zum Austragen des Behältnisinhalts (2). Der Behältnisinhalt (2) ist in einem Zylinder (1) einer Kolben-Zylinder-Anordnung (7, 1) befindlich, wobei die dem Kolben (7) gegenüberliegende Stirnseite des Zylinders (1) durch eine Membran (4) abgeschlossen ist. Der Kolben (7) weist eine zur Membran (4) hin gerichtete Spitze (8) sowie einen Kanal (13) auf, über den nach Eindringen der Spitze (8) durch die Membran (4) hindurch der Inhalt (2) des Behältnisses aus dem Zylinder (1) heraus zu einer Entnahmestelle befördert wird.

## Beschreibung

Die Erfindung betrifft eine Vorrichtung gemäß den im Oberbegriff des Anspruchs 1 angegebenen Merkmalen.

Insbesondere im ärztlichen und zahnärztlichen Bereich sind derartige Vorrichtungen bekannt, aber auch für andere Anwendungen. Im zahnärztlichen Bereich ist eine solche Vorrichtung beispielsweise als Carpul-System bekannt. Diese Vorrichtung besteht aus einem ein Arzneimittel, beispielsweise ein Betäubungsmittel, aufnehmenden Behältnis in Form einer Ampulle, einer Halterung zur Aufnahme dieser Ampulle und einer Kanüle. Die Ampulle besteht aus Glas, ist an einem Ende durch einen darin befindlichen Kolben und am anderen durch eine Gummimembran verschlossen. Zum Gebrauch wird die Ampulle in die dafür vorgesehene Halterung eingelegt, eine halterungsseitig verschiebbar gelagerte Stange mit dem Kolben verbunden sowie eine ebenfalls in die Halterung eingelegte Kanüle mit dem Inneren der Ampulle verbunden, indem das proximale Ende durch die Gummimembran gedrückt wird. Bei diesem System werden die Ampulle und die Kanüle nach Gebrauch ersetzt, die Halterung ist zum regelmäßigen Wiedergebrauch vorgesehen.

Zwar hat sich dieses System in dem vorbeschriebenen speziellen Einsatzbereich bewährt, doch ist es vergleichsweise aufwändig und hat darüber hinaus auch Handhabungsnachteile.

Die zur ständigen Benutzung vorgesehene Halterung ist teuer und somit nur dort wirtschaftlich einsetzbar, wo die Vorrichtung regelmäßig benutzt wird. Auch die Herstellung der aus Glas bestehenden Ampulle mit dem darin befindlichen Kolben und der Gummimembran am anderen Ende ist aufwändig, im Übrigen nur für gut fließfähige Materialien geeignet. Darüber hinaus ist die Handhabung vergleichsweise kompliziert, es muss nämlich die Ampulle und die Kanüle an der vorgesehenen Stelle in die Halterung eingelegt werden, sodann muss die Stange durch Schrauben mit dem ampullenseitigen Kolben verbunden werden und die Kanüle ebenfalls durch Schrauben durch die Membran der Ampulle gedrückt werden.

Darüber hinaus ist es bekannt, Medikamente in Glasampullen abzufüllen. Diese hermetisch abgeschlossenen Glasampullen sind an einer Soll-Bruchstelle aufzubrechen, wonach der darin befindliche Stoff entnommen werden kann. Derartige Behältnisse sind nur für dünnflüssige Stoffe geeignet, diese dürfen darüber hinaus nicht leicht entzündlich oder explosionsgefährdet sein. Die Handhabung solcher Glasampullen ist zudem aufwändig. Abgesehen von der Verletzungsgefahr beim Aufbrechen und der Gefahr, dass winzige Glaspartikel in das Behältnis gelangen, bedingt die Entnahme des Behältnisinhalts stets mehrere Schritte, die in der Regel nur mit beiden Händen ausgeführt werden können.

Vor diesem Hintergrund liegt der Erfindung die Aufgabe zugrunde, eine gattungsgemäße Vorrichtung zu schaffen, die einfach in der Anwendung und kostengünstig in der Herstellung ist, insbesondere die vorerwähnten Nachteile vermeidet.

Diese Aufgabe wird gemäß der Erfindung durch die in Anspruch 1 angegebenen Merkmale gelöst. Vorteilhafte Ausgestaltungen der Erfindung sind in den Unteransprüchen, der nachfolgenden Beschreibung sowie der Zeichnung angegeben.

Erfindungsgemäß ist das Behältnis, dessen Inhalt eine Flüssigkeit, ein Gas oder eine Paste sein kann, durch einen Zylinder einer Kolben-Zylinder-Anordnung gebildet, wobei unter Zylinder im Sinne der Erfindung nicht ein geometrischer, sondern ein hydraulischer im Sinne einer Kolben-Zylinder-Anordnung zu verstehen ist. Es kommt also nicht auf die Querschnittsform des Zylinders an, sondern lediglich darauf, dass darin ein Kolben verfahrbar ist. Ein Zylinder gemäß der Erfindung kann somit im Querschnitt auch oval oder eckig sein, wenn dies erforderlich oder auch nur zweckmäßig sein sollte. Dieser das Behältnis bildende Zylinder ist topfartig ausgebildet, weist also einen Boden und eine Zylinderwandung auf und ist an einer Stirnseite membranartig abgeschlossen. Diese Membran wird nach Befüllen des Behältnisses aufgebracht und schließt dieses mit dem darin befindlichen Inhalt hermetisch ab. Dem das Behältnis bildenden Zylinder ist ein Kolben zugeordnet, der eine Spitze aufweist sowie einen Kanal, über den nach Eindringen der Spitze durch die Membran hindurch in den Zylinder der Austrag des Behältnisinhalts erfolgt. Dabei ist die Vorrichtung bevorzugt so angeordnet, dass Kolben und Zylinder dieser Kolben-Zylinder-Anordnung gebrauchsfertig einander zugeordnet sind, so dass allein durch Zusammenschieben der vorgenannten Bauteile ein Austrag des im Zylinder befindlichen Mittels durch den im Kolben vorgesehenen Kanal erfolgt. Die Handhabung ist somit besonders einfach, da das Öffnen des Behältnisses und der anschließende Austrag ohne eine Änderung in der Handhabung der Vorrichtung mit nur einer Bewegung der Bedienperson erfolgen können.

Die erfindungsgemäße Vorrichtung eignet sich daher prinzipiell für eine Vielzahl von Anwendungen nicht nur im medizinischen Bereich. Es kann mit der erfindungsgemäßen Vorrichtung eine sofort gebrauchsfertige medikamentengefüllte Spritze gebildet werden, wobei auch Anforderungen an Keimfreiheit systembedingt einfach und zuverlässig erfüllt werden können. Dabei wird die Vorrichtung bevorzugt aus Spritzgussteilen, vorzugsweise einem Kolben und einem zylinderseitigen Spritzgussteil gebildet sein, die kostengünstig hergestellt und somit zum Einmalgebrauch vorgesehen sein können.

Um den Kolben in einer Position zu halten, in der er durch Einschieben zunächst die Membran durchdringt und dann das im Zylinder befindliche Mittel über seinen Kanal austrägt, kann entweder der Zylinder über die Membran hinaus ganz oder teilweise verlängert sein, um den Kolben zu tragen, oder aber, was zu bevorzugen ist, kolbenseitig eine Führung vorgesehen, in die der Zylinder einschiebbar ist. Das Vorsehen einer solchen kolbenseitigen Führung hat den Vorteil, dass die Vorrichtung ähnlich einer üblichen Spritze gehandhabt werden kann, also ein gut dosierbarer Austrag erreicht wird.

Zweckmäßigerweise wird diese Führung in Form eines Führungszylinders ausgebildet, an dessen Boden der Kolben fest angebracht ist. Dabei weist die Spitze des Kolbens vom Boden des Führungszylinders weg in Richtung auf die Membran. Der das Behältnis bildende Zylinder sitzt dann innerhalb dieses Führungszylinders kolbenartig, wobei zum Durchdringen der Membran und Entleeren des Behältnisses der das Behältnis bildende Zylinder in den Führungszylinder eingeschoben wird. Dadurch schiebt sich auch der im Boden des Führungszylinders befestigte Kolben in den das Behältnis bildenden Zylinder, wodurch zunächst die Membran durchstochen und dann der Austrag des Behälterinhalts durch den durch den Kolben verlaufenden Kanal erfolgt.

Der Führungszylinder kann an seinem der Kolbenspitze abgewandten Ende praktisch beliebig ausgebildet und somit in idealer Weise an den jeweiligen Anwendungszweck angepasst sein. So kann beispielsweise ein Luer- oder Luerlockanschluss vorgesehen sein, es kann auch eine langgestreckte rohrförmige Spitze angeformt sein, um das im Behältnis befindliche Mittel direkt in enge Hohlräume, beispielsweise Zahnwurzelkanäle und dergleichen, führen zu können. So können am Ende auch pinselartige Gebilde oder andere Austragshilfen vorgesehen sein. Wenn das Mittel nicht zum direkten Aufbringen vorgesehen ist, so kann statt einer Austragsspitze auch ein nach oben offenes Entnahmegefäß vorgesehen sein, in das ein Pinsel oder anderes Instrument eintauchbar ist.

Bei entsprechender Ausbildung des Kolbens sowie des Zylinderbodens kann das Restvolumen innerhalb der Vorrichtung nach dem Entleeren auf ein Minimum reduziert werden. Hierzu ist der Boden des das Behältnis bildenden Zylinders entsprechend der Kolbenspitze auszuformen, darüber hinaus muss der Kolben mindestens so lang sein wie der Zylinder, damit die Kolbenspitze bis zum Boden des Zylinders eingeschoben werden kann.

Der Zylinder, der das Behältnis bildet, wird befüllt und mit einer Membran, vorzugsweise einer Folie, z. B. einer mit Kunststoff beschichteten Metallfolie, abgeschlossen, wobei zweckmäßigerweise eine thermische Verschweißung zwischen der freien Stirnseite des Zylinders und der Folie erfolgt. Alternativ kann auch eine Klebung, Bördelung oder andere geeignete Verbindung hergestellt werden. Wenn sich die Membran über den gesamten Zylinderquerschnitt erstreckt, ist dies vorteilhaft im Hinblick auf die Herstellung des Zylinders einerseits und die Füllung desselben andererseits. So können auch pastöse Stoffe in das Behältnis gefüllt und mit der Vorrichtung ausgetragen werden.

Ein weiterer wesentlicher Aspekt der vorliegenden Erfindung liegt darin, dass die Vorrichtung nicht nur mit einem, sondern auch mit zwei, drei oder mehr zylinderartigen Behältnissen versehen sein kann, deren Kanäle zusammengeführt sind. Es kann also hiermit beispielsweise ein Zwei-Komponenten-Kleber sofort gebrauchsfertig im richtigen Mischungsverhältnis ausgetragen werden. Es können Stoffe unterschiedlicher Viskosität miteinander gemischt werden. Das Mischungsverhältnis kann konstruktiv variiert werden. So können beispielsweise zwei Kolben-Zylinder-Anordnungen nebeneinander vorgesehen sein, wobei die Bauteile kolben- bzw. zylinderseitig fest miteinander verbunden sind, so dass beim Einschieben zunächst die beiden nebeneinander liegenden Kolbenspitzen die Membranen der fluchtend dazu angeordneten Zylinder durchstoßen und dann in diese eindringen. Die Kanäle der Kolben können zusammengeführt oder beispielsweise in einem gemeinsamen Mischgefäß münden, so dass die Vorrichtung aus zwei Spritzgussteilen aufgebaut werden kann.

In einer Weiterbildung der Erfindung können weitere Kolben-Zylinder-Anordnungen vorgesehen sein, um wahlweise das in einem Behältnis befindliche Mittel mit dem eines anderen oder eines dritten zu mischen, beispielsweise um einen schnell oder langsam härtenden Zwei-Komponenten-Kunststoff zu mischen oder um unterschiedliche Mischungsverhältnisse zu realisieren.

Um sicherzustellen, dass der Inhalt der alternativ in Frage kommenden Behältnisse nicht gleichzeitig zugemischt wird, ist gemäß einer Weiterbildung der Erfindung eine mechanische Sperre vorgesehen, welche die Mittel zum Austragen des anderen Behältnisses sperrt, wenn der Austrag aus einem weiteren Behältnis erfolgt oder umgekehrt. Wie die vorstehenden Ausführungen verdeutlichen, ist die erfindungsgemäße Vorrichtung sowohl von der Anwendung als auch von der konstruktiven Varianz her extrem vielseitig. Die Vorrichtung kann beispielsweise eingesetzt werden für Ein- oder Zwei-Komponenten-Klebstoffe, für Ätzmittel, für Primer, für Versiegelungslacke, für Spüllösungen für Wurzelkanalbehandlungen, für Füllstoffe, für Zemente oder für Medikamente, um nur einige Anwendungsgebiete beispielhaft zu nennen.

Die Erfindung ist nachfolgend anhand von in der Zeichnung dargestellten Ausführungsbeispielen im Einzelnen erläutert. Es zeigen:
- Fig. 1: in vereinfachter Darstellung eine erste Ausführung einer erfindungsgemäßen Vorrichtung vor dem Austragen im Schnitt,
- Fig. 2: die Vorrichtung gemäß Fig. 1 nach dem Austragen in Darstellung nach Fig. 1,
- Fig. 3: ein zweites Ausrührungsbeispiel in Darstellung nach Fig. 1,
- Fig. 4: ein drittes Ausführungsbeispiel in Darstellung nach Fig. 1,
- Fig. 5: ein viertes Ausführungsbeispiel in Darstellung nach Fig. 1,
- Fig. 6: das Ausführungsbeispiel nach Fig. 5 in Darstellung nach Fig. 2,
- Fig. 7: ein fünftes Ausführungsbeispiel in Darstellung nach Fig. 1,
- Fig. 8: das Ausführungsbeispiel gemäß Fig. 7 in Darstellung nach Fig. 2,
- Fig. 9: ein sechstes Ausführungsbeispiel in Darstellung nach Fig. 1,
- Fig. 10: das Ausführungsbeispiel gemäß Fig. 9 in Darstellung gemäß Fig. 2,
- Fig. 11: ein siebtes Ausführungsbeispiel in Darstellung nach Fig. 1,
- Fig. 12: das Ausführungsbeispiel gemäß Fig. 11 in einer ersten Variante gemäß Darstellung nach Fig. 2 und
- Fig. 13: in einer zweiten Variante in Darstellung nach Fig. 2.

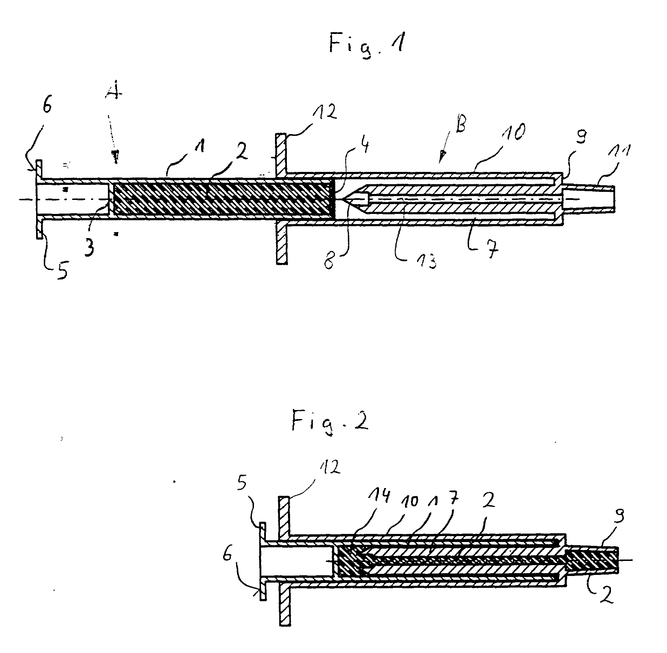

Die anhand von Fig. 1 dargestellte Vorrichtung weist eine Kolben-Zylinder-Anordnung auf, deren Zylinder 1 ein Behältnis für eine Flüssigkeit 2 bildet. Der Zylinder 1 weist einen Zylinderboden 3 auf und ist an der dem Zylinderboden 3 gegenüberliegenden Seite durch eine Membran 4 in Form einer kunststoffbeschichteten Folie hermetisch abgeschlossen. Die Zylinderwandung 1 ist über den Boden 3 hinaus verlängert und am freien Ende durch einen Flansch 5 erweitert, der eine Auflagefläche 6 zur Handhabung der Vorrichtung bildet.

Ein Kolben 7 ist achsgleich zum Zylinder 1 angeordnet und bildet den anderen Teil der Kolben-Zylinder-Anordnung. Der Kolben 7 ist an seinem zur Membran 4 weisenden Ende mit einer Spitze 8 versehen, die so ausgebildet ist, dass bei Überschreiten einer vorbestimmten Anpresskraft die Spitze 8 die Membran 4 durchbricht und in das Innere des Zylinders 1, also des Behältnisses, eindringt.

Bei der dargestellten Ausführung sind Zylinder 1 und Kolben 7 im Querschnitt rund ausgebildet und größenmäßig so aufeinander abgestimmt, dass der Kolben 7 mit seinem Außenumfang im Wesentlichen ohne Spiel in den Innenumfang des Zylinders 1 unter Verdrängung der darin befindlichen Flüssigkeit 2 einfahren kann.

Der Kolben 7 ist am Boden 9 eines Führungszylinders 10 angeformt, der kreisrunden Querschnitt hat und vom Innendurchmesser so ausgebildet ist, dass der Zylinder 1 im Wesentlichen spielfrei in diese Führung 10 nach Art eines Kolbens einschiebbar ist. Der Führungszylinder 10 ist - abgesehen vom Kolben 7 - wie ein Spritzenzylinder aufgebaut, d. h. am Boden 9 ist ein nach außen gerichteter, konisch zulaufender Rohrabschnitt als Lueranschluss angeformt. Am anderen Ende ist ein Flansch 12 angeformt, der eine Handhabe in Verbindung mit der Auflagefläche 6 bildet.

Der Kolben 7 weist einen zentralen Kanal 13 auf, über den die im Zylinder 1 befindliche Flüssigkeit nach Durchdringen der Membran 4 mit der Kolbenspitze 8 entweicht. Der Kanal 13 schließt endseitig an den Lueranschluss 11 an, so dass beim Zusammenschieben der Kolben-Zylinder-Anordnung die im Zylinder 1 (Behältnis) befindliche Flüssigkeit über den Kanal 13 zum Lueranschluss 11 geführt wird, an den beispielsweise eine übliche Spritzenkanüle aufsteckbar ist.

Bei der Darstellung nach Fig. 2 ist der Kolben 7 vollständig in den Zylinder 1 eingefahren, das Behältnis also soweit wie möglich entleert. Bei entsprechender Ausgestaltung des Kolbens 7 und des Zylinderbodens 3 kann das in Fig. 2 im Zylinder 1 noch befindliche Restvolumen 14 auf ein Minimum reduziert werden. Hierzu wird der Zylinderboden 3 entsprechend der Kolbenspitze 8 konturiert und der Kolben 7 längenmäßig so dimensioniert, dass er in eingeschobenem Zustand (Fig. 2) den Zylinder 1 über seine ganze Länge ausfüllt.

Die anhand der Figuren 1 und 2 dargestellte Vorrichtung ist nach Art einer Spritze handhabbar, wobei die Bedienperson, ohne abzusetzen, die Vorrichtung aus der in Fig.1 dargestellten Stellung, bei der die Flüssigkeit 2 innerhalb des Zylinders 1 durch die Membran 4 hermetisch nach außen hin abgeschlossen ist, in die in Fig. 2 dargestellte Stellung verbracht wird, in der die Flüssigkeit 2 nahezu vollständig aus dem Behältnis 1 ausgetragen worden ist.

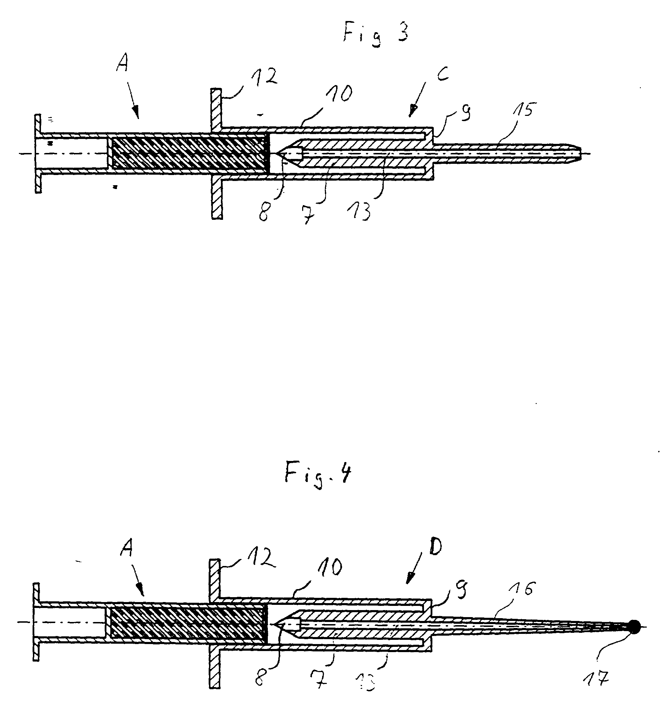

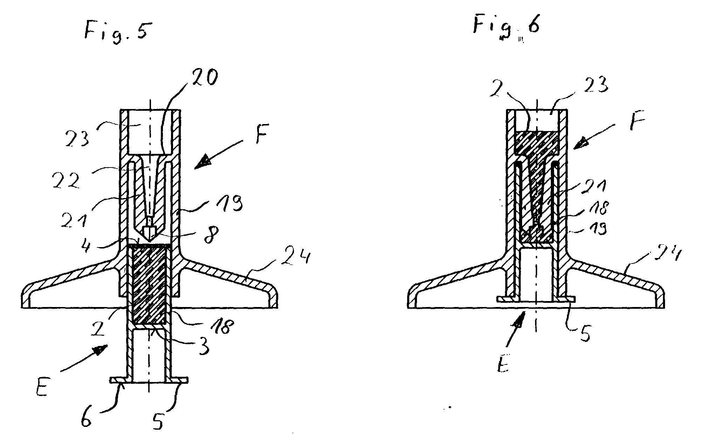

Die anhand der Figuren 1 und 2 dargestellte Vorrichtung besteht aus zwei Spritzgussteilen A und B und ist zum einmaligen Gebrauch bestimmt. Es kann jedoch auch der Zylinder wiederbefüllt und erneut durch eine Folie verschlossen werden. Auch ist das Spritzgussteil A mit anderen Spritzgussteilen C oder D verwendbar, die anhand der Figuren 3 und 4 beispielhaft dargestellt sind und eine Anpassung des kolbenseitigen Bauteils im Hinblick auf den jeweiligen Anwendungsbereich zeigen.

Die anhand von Fig. 3 dargestellte Ausführungsvariante unterscheidet sich von der anhand von Fig. 1 dargestellten lediglich dadurch, dass anstelle des Lueranschlusses 11 ein langgestrecktes dünnes Rohr 15 vorgesehen ist, durch welches der Austrag der im Behälter 1 befindlichen Flüssigkeit erfolgt. Durch solch ein Rohr 15 können beispielsweise Zahnhohlräume oder Bohrungen zielsicher erreicht werden.

Bei der Ausführungsvariante nach Fig. 4 ist anstelle des Rohrs 15 eine konisch zulaufende Hohlspitze 16 vorgesehen, an deren Ende eine mit Öffnungen versehene beflockte Kugel 17 angeordnet ist, durch welche die Flüssigkeit 2 ausgetragen wird und mit der diese gleichzeitig verteilt werden kann.

Die anhand der Figuren 5 und 6 dargestellte Ausführungsvariante besteht aus einem das Behältnis aufnehmenden Spritzgussteil E sowie einem den Kolben und die Führung bildenden Spritzgussteil F. In Funktion und Aufbau entspricht das Spritzgussteil E dem Spritzgussteil A der Figuren 1 bis 4, es weist lediglich eine geringere Länge auf, weshalb der Zylinder hier mit 18 gekennzeichnet ist. Auch bei dieser Ausführungsvariante ist der Zylinder 18 über den Zylinderboden 3 hinaus verlängert und an seinem Ende mit einem Flansch 5 versehen, dessen Auflagefläche 6 zum Handhaben, insbesondere zum Einschieben des Zylinders 18 in das Bauteil F, dient.

Das Spritzgussteil F weist einen Führungszylinder 19 auf, an dessen Boden 20 ein Kolben 21 angeordnet ist, der sich in Richtung auf den Zylinder 18 erstreckt. Der Kolben 21 weist eine Spitze 8 auf, welche zum Durchdringen der Membran 4 vorgesehen ist, mit welcher die im Zylinder 18 befindliche Flüssigkeit 2 hermetisch abgeschlossen ist. Der Kanal 22 des Kolbens 21 weitet sich von der Spitze 8 zum Boden 20 hin konisch auf und mündet dort in einem topfartigen Entnahmegefäß 23, das durch Verlängerung des Führungszylinders 19 über den Boden 20 hinaus gebildet ist.

Weiterhin weist der Führungszylinder 19 einen tellerartigen Flansch 24 als Handhabe und Fuß auf. Zum Gebrauch der im Zylinder 18 befindlichen Flüssigkeit 2 wird das Spritzgussteil E vollständig in den Führungszylinder 19 eingeschoben, bis die in Fig. 6 dargestellte Stellung erreicht ist. Dann ist der Zylinder 18 durch den Kolben 21 über den Kolbenkanal 22 nahezu vollständig entleert. Die Flüssigkeit befindet sich in dem nach oben offenen Entnahmegefäß 23. Der tellerartige Flansch 24 überragt nun den Flansch 5, so dass die gesamte Vorrichtung auf diesem abgestellt werden kann, der Flansch 24 somit als Fuß dient. Dann kann die im Entnahmegefäß 23 befindliche Flüssigkeit mit einem Pinsel oder einem Auftragsmittel bedarfsweise entnommen werden. Die Vorrichtung kann also in einem Arbeitsgang geöffnet und zur Entnahme der Flüssigkeit bereitgestellt werden. Diese Tätigkeit kann mit nur einer Hand erfolgen.

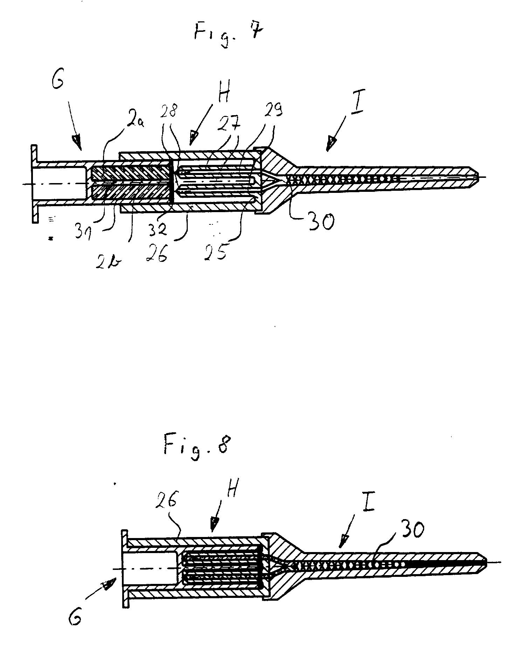

Die Ausführungsvariante nach den Figuren 7 und 8 besteht aus drei Spritzgussbauteilen G, H und I. Das Bauteil G entspricht den Bauteilen A bzw. E der vorbeschriebenen Ausführungsvarianten, die Bauteile H und I im Wesentlichen den Bauteilen C bzw. D. Im Unterschied zu den vorbeschriebenen Ausführungsvarianten sind bei der Ausführung nach Fig. 7 und 8 zwei Kolben-Zylinder-Anordnungen nebeneinander vorgesehen. Hierzu sind im Boden 25 eines Führungszylinders 26 zwei Kolben 27 angeordnet, die ebenfalls je eine Spitze 28 sowie je einen zentralen Kanal 29 aufweisen. Auf das den Kolben 27 abgewandte Ende des Führungszylinders 7 ist das Bauteil I aufgesetzt, in welchem die beiden Kolbenkanäle 29 in einem gemeinsamen Austragskanal 30 zusammengeführt sind. Innerhalb des Kanals 30 sind (nicht im Einzelnen dargestellte) Schikanen (ein statischer Mischer) vorgesehen, welche für eine intensive Durchmischung der aus den beiden Kanälen 29 kommenden Flüssigkeiten sorgt, so dass das fertig gemischte Produkt am Ende des Austragskanals 30 zur Verfügung steht.

Entsprechend den beiden Kolben 27 sind innerhalb des Bauteils G zwei Zylinder 31 vorgesehen, die durch eine gemeinsame Membran 32 verschlossen sind. Innerhalb der Zylinder 31 sind Flüssigkeiten 2a und 2b eingeschlossen. Die aus den Zylindern 31 und Kolben 27 bestehenden Kolben-Zylinder-Anordnungen haben jeweils kreisrunden, aufeinander abgestimmten Querschnitt. Der Führungszylinder 6 ist im Querschnitt oval abgeflacht ebenso wie das darin einschiebbare Bauteil G, um eine definierte Führung beim Einfahren der Kolben 27 in die Zylinder 31 zu bilden. Es werden also beim Einfahren des Bauteils G in das Bauteil H die in den Zylindern 31 befindlichen Flüssigkeiten 2a und 2b über die Kanäle 29 in den Austragskanal 30 gepresst, wo sie intensiv vermischt werden und an dessen Ende sie ins Freie gelangen.

Fig. 8 zeigt die Vorrichtung gemäß Fig. 7 in eingefahrener Stellung, in welcher die Behältnisse mit den Flüssigkeiten 2a und 2b nahezu vollständig entleert sind.

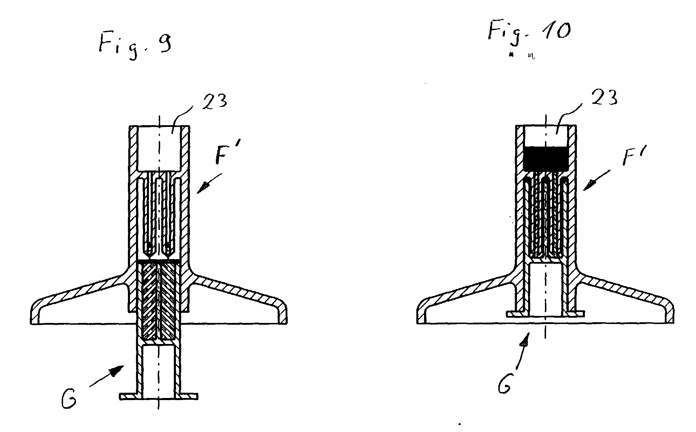

Die anhand der Figuren 9 und 10 dargestellte Ausführungsvariante entspricht hinsichtlich der Doppel-Kolben-Zylinder-Anordnung der anhand der Figuren 7 und 8 vorbeschriebenen und ist im Übrigen entsprechend der anhand der Figuren 5 und 6 beschriebenen ausgebildet, wobei anstelle einer Austragsspitze ein Entnahmegefäß 23 vorgesehen ist, in dem in eingeschobenem Zustand das fertig gemischte, aus den Flüssigkeiten 2a und 2b bestehende Produkt zur Verarbeitung ansteht. Das Bauteil G der Figuren 9 und 10 ist identisch mit dem anhand der Figuren 7 und 8 beschriebenen. Das Bauteil F' entspricht im Wesentlichen dem anhand der Figuren 5 und 6 beschriebenen Bauteil F, wobei dort nicht ein, sondern zwei Kolben passend zu den Doppel-Zylindern 31 vorgesehen sind.

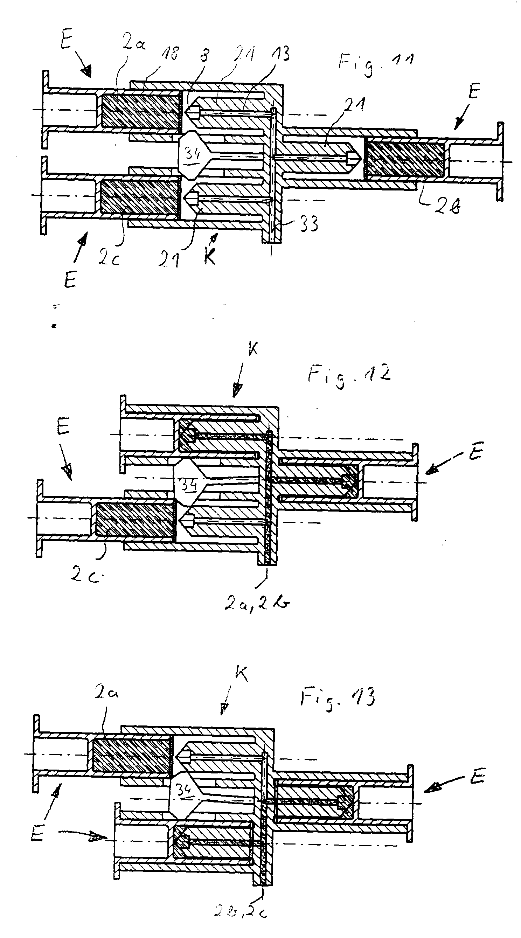

Bei der anhand der Figuren 11 bis 13 dargestellten Vorrichtung sind drei Bauteile E, so wie sie anhand der Figuren 5 und 6 beschrieben worden sind, vorgesehen, die in einem gemeinsamen Führungsbauteil K angeordnet sind. Die behältnisbildenden Bauteile E sind mit Flüssigkeiten 2a, 2b und 2c gefüllt, wobei die Flüssigkeit 2b wahlweise mit der Flüssigkeit 2a oder mit der Flüssigkeit 2c gemischt werden kann. Hierzu ist jedem der behältnisbildenden Zylinder 18 ein Kolben 21 mit Spitze 8 und einem zentralen Kanal 13 zugeordnet. Die Kanäle 13 der drei Kolben 21 münden in einem gemeinsamen Querkanal 33, der den Austragskanal der Vorrichtung bildet. Wie die Figuren zeigen, sind zwei Kolben links des Kanals 33 und einer rechts des Kanals 33 angeordnet, entsprechend die zugehörigen Bauteile E. Zum Austragen und Mischen der Flüssigkeiten 2a und 2b bzw. 2c und 2b sind die entsprechenden Bauteile E in das Bauteil K, welches die Führung für alle Bauteile E bildet und die Kolben 21 trägt, einzuschieben. Dabei ist zwischen den nebeneinander angeordneten Zylindern 18 ein Sperrglied 34 derart bewegbar angeordnet, dass nach dem anfänglichen Einschieben einen linken Zylinders dieses Sperrglied 34 in den Weg des anderen Zylinders geschoben wird und diesen damit blockiert sowie auch umgekehrt. Damit wird sichergestellt, dass die Flüssigkeit 2b stets nur mit der Flüssigkeit 2a oder mit der Flüssigkeit 2c, nicht jedoch mit beiden Flüssigkeiten, gemischt wird.

Die Fig. 12 zeigt die Stellung, in der die Mischung der Flüssigkeiten 2a und 2b erfolgt ist, wohingegen die Fig. 13 die Stellung zeigt, in der eine Mischung der Flüssigkeiten 2c und 2b erfolgt ist. Deutlich sichtbar ist, dass das Sperrglied 34 in Fig. 12 den unteren, die Flüssigkeit 2c beinhaltenden Zylinder 18 und in Fig. 13 den oberen, die Flüssigkeit 2a beinhaltenden Zylinder in seiner Stellung verriegelt.

### Bezugszeichenliste

- 1 -: Zylinder, Behältnis
- 2 -: Flüssigkeit
- 3 -: Zylinderboden
- 4 -: Membran
- 5 -: Flansch
- 6 -: Auflagefläche
- 7 -: Kolben
- 8 -: Spitze
- 9 -: Boden
- 10 -: Führungszylinder
- 11 -: Lueranschluss
- 12 -: Flansch
- 13 -: Kanal
- 14 -: Restvolumen
- 15 -: Rohr
- 16 -: Hohlspitze
- 17 -: beflockte Kugel
- 18 -: Zylinder
- 19 -: Führungszylinder
- 20 -: Boden
- 21 -: Kolben
- 22 -: Kanal
- 23 -: Entnahmegefäß
- 24 -: Tellerflansch, Fuß
- 25 -: Boden
- 26 -: Führungszylinder
- 27 -: Kolben
- 28 -: Spitze

- 29 -: Kanal
- 30 -: Austragskanal
- 31 -: Zylinder
- 32 -: Membran
- 33 -: Querkanal
- 34 -: Sperrglied

- A,B -: Spritzgussteile
(Fig. 1 und 2)
- C -: Spritzgussteil (Fig. 3)
- D -: Spritzgussteil (Fig. 4)
- E -: Spritzgussteile
(Fig. 5, 6, 11, 12, 13)
- F -: Spritzgussteil
(Fig. 5, 6)
- G,H,I -: Spritzgussteile
(Fig. 7, 8)
- G, F' -: Spritzgussteile
(Fig. 9, 10)
- K -: Spritzgussteil
(Fig. 11, 12, 13)

## Patentansprüche

1. Vorrichtung mit einem dicht abgeschlossenen, flüssigkeits-, pasten- oder gasgefüllten Behältnis (1) und mit Mitteln zum Austragen des Behältnisinhalts (2), **dadurch gekennzeichnet, dass** das Behältnis durch einen Zylinder (1) einer Kolben-Zylinder-Anordnung (7, 1) gebildet ist, dass eine Stirnseite des Zylinders (1) membranartig (4) abgeschlossen ist und dass der Kolben (7) eine zur Membran (4) hin gerichtete Spitze (8) sowie einen Kanal (13) aufweist, über den nach Eindringen der Spitze (8) durch die Membran hindurch in den Zylinder der Austrag des Behältnisinhalts (2) erfolgt.

2. Vorrichtung nach Anspruch 1, **dadurch gekennzeichnet, dass** der Kolben (7) Teil einer Führung (10) ist, in die der Zylinder (1) einschiebbar ist.

3. Vorrichtung nach einem der vorhergehenden Ansprüche, **dadurch gekennzeichnet, dass** die Führung durch einen Führungszylinder (10) gebildet ist und dass sich der Kolben (7) vom Boden (9) des Führungszylinders (10) in Richtung zu dem das Behältnis bildenden Zylinder (1) erstreckt.

4. Vorrichtung nach einem der vorhergehenden Ansprüche, **dadurch gekennzeichnet, dass** die Vorrichtung spritzenartig ausgebildet ist, wobei der das Behältnis bildende Zylinder (1) den Spritzenkolben und der Führungszylinder (10) den Spritzenzylinder bildet.

5. Vorrichtung nach einem der vorhergehenden Ansprüche, **dadurch gekennzeichnet, dass** zwei oder mehr Kolben-Zylinder-Anordnungen (27, 31) mit membranartig abgeschlossenen Zylindern (31) als Behältnisse und damit in Wirkverbindung stehenden Kolben (27) vorgesehen sind, wobei die Kanäle (29) der Kolben (27) zum Zwecke des Mischens des Inhalts der zwei oder mehr Behältnisse (31) zusammengeführt sind.

6. Vorrichtung nach einem der vorhergehenden Ansprüche, **dadurch gekennzeichnet, dass** der Kanal (22) des Kolbens (21) oder die Kanäle (29) der Kolben (27) in einem einseitig offenen Mischgefäß (23) oder einer gemeinsamen Austragsspitze (30) münden.

7. Vorrichtung nach einem der vorhergehenden Ansprüche, **dadurch gekennzeichnet, dass** die Vorrichtung aus vorzugsweise zwei Kunststoffspritzgussteilen (A, B) gebildet ist, wobei ein Teil (A) ein oder mehrere Behältnisse (1) und ein anderes Teil (B) die Führung (10) sowie einen oder mehrere Kolben (7) umfasst.

8. Vorrichtung nach einem der vorhergehenden Ansprüche, **dadurch gekennzeichnet, dass** ein ein Behältnis bildender Zylinder (1) nach dem Befüllen stirnseitig mit einer Folie (4) abgedeckt und dicht verschweißt ist.

9. Vorrichtung nach einem der vorhergehenden Ansprüche, **dadurch gekennzeichnet, dass** zwei oder mehr Kolben-Zylinder-Anordnungen (27, 31) nebeneinander in einer gemeinsamen Führung, vorzugsweise in einem gemeinsamen Führungszylinder (26), angeordnet sind.

10. Vorrichtung nach einem der vorhergehenden Ansprüche, **dadurch gekennzeichnet, dass** drei oder mehr Kolben-Zylinder-Anordnungen (18, 21) mit membranartig abgeschlossenen Zylindern (18) als Behältnisse vorgesehen sind, wobei der Inhalt (2b) des einen Behältnisses wahlweise mit dem (2a) des anderen oder mit dem (2c) eines weiteren Behältnisses beim Austragen mischbar ist, **dadurch gekennzeichnet, dass** eine mechanische Sperre (34) vorgesehen ist, welche die Mittel zum Austragen des Inhalts des anderen Behältnisses sperrt, wenn der Austrag aus dem weiteren Behältnis erfolgt und umgekehrt.
